# EUROPEAN PATENT SPECIFICATION

(11) **EP 4 284 451 B1**
(45) Date of publication and mention of the grant of the patent: **30.07.2025**
(21) Application number: 21787036.9
(22) Date of filing: 16.09.2021
(51) Int. Cl.: A61L 2/20, A61L 9/015, A61L 9/12, F24F 8/26, B05B 14/00

(54) **OZONE SANITIZING DEVICE**
OZONDESINFEKTIONSVORRICHTUNG
DISPOSITIF DE DÉSINFECTION À L'OZONE

(30) Priority: 26.01.2021 IT 202100001514
(43) Date of publication of application: 06.12.2023
(73) Proprietor: Solidea S.r.l., 41051 Castelnuovo Rangone (MO) (IT)
(72) Inventor: VENTURI, Marco, 41051 Castelnuovo Rangone (MO) (IT)
(74) Representative: Brunacci, Marco
(86) International application number: PCT/IB2021/058451
(87) International publication number: WO 2022/162449

(56) References cited:
- DE-U1- 202020 106 640
- JP-A- H11 226 108
- US-A1- 2004 003 511
- US-A1- 2021 015 953
- ANONYMOUS: "Aeria il sistema di purificazione che lava l'aria senza uso di filtri", 31 March 2021 (2021-03-31), pages 1 - 18, XP055848022, Retrieved from the Internet <URL:https://www.aeria.bio> [retrieved on 20211005]

## Description

### Technical Field

The present invention relates to an ozone sanitizing device.

### Background Art

The present invention finds a preferred, but not exclusive, application in the disinfection of the air of closed environments, in particular those which are very crowded, since they are usually characterized by the presence of a particulate matter rich in polluting agents, such as, e.g., dusts, odors of different types, fumes, unburned hydrocarbons, bacteria, viruses, fungi, carbon oxide, etc. Generally, the sanitizing devices are provided with a system for the introduction of ozone into the environment.

It is known, in fact, that ozone, through its interaction with viral and bacterial particles present in the air or through contact with surfaces placed in the environment, is able to exert an antiviral and antibacterial action.

Ozone disinfection can be carried out by several mechanisms.

A first mechanism involves the use of a hydraulic circuit which takes air from the environment, mixes the air taken with ozone and, subsequently, introduces the mixed air into the environment.

**In** a second mechanism, on the other end, the hydraulic circuit releases the ozone directly into the environment.

In both cases, on completion of the disinfection treatment, the ambient air is contaminated by the presence of a high concentration of ozone which can have harmful effects on human health. It is therefore usual to air the room for a certain period of time in order to reduce the concentration of ozone in the air.

However, this solution is not completely satisfactory as it usually requires an extremely long resting time to bring the ozone concentration in the air back to an acceptable level.

Moreover, this solution is most of the times not very effective. **In** fact, there can be situations in which it is difficult to ventilate the environment, for example due to the lack or limited presence of windows and/or ventilation systems.

Ozone sanitizing devices of the known type are described in documents DE2020106640U1,US2021015953A1, JPH11226108A and US2004/003511A1.

### Description of the Invention

Therefore, the main aim of the present invention is to devise an ozone sanitizing device able to sanitize the air of a room quickly, fast and effectively.

A further object of the present invention is to devise a sanitizing device able to rapidly reduce the concentration of ozone present in the air of a disinfected room.

Another object of the present invention is to devise a versatile, small-sized, low-cost device.

Another object of the present invention is to devise a sanitizing device allowing to overcome the mentioned drawbacks of the prior art within a simple, rational, easy and effective to use as well as affordable solution.

The aforementioned objects are achieved by the present ozone sanitizing device having the characteristics of claim 1.

### Brief Description of the Drawings

Other characteristics and advantages of the present invention will become more apparent from the description of a preferred, but not exclusive, embodiment of an ozone sanitizing device, illustrated by way of an indicative, yet non limiting example, in the accompanying tables of drawings wherein:
- Figure 1 is a perspective view from above of the sanitizing device according to the present invention,
- Figure 2 is a perspective view from above of the device in Figure 1 where the casing has been removed for better clarity,
- Figure 3 is a sectional view of the sanitizing means of the device in Figure 1,
- Figure 4 is a sectional view of the deozonization means of the device in Figure 1.

### Embodiments of the Invention

With particular reference to such figures, reference numeral 1 globally indicates an ozone sanitizing device.

Suitably, the device 1 comprises a supporting frame 2 positionable inside a room to be sanitized. Furthermore, the device 1 comprises ozone sanitizing means 3 mounted on the supporting frame 2 and configured to release ozone into the room.

Ozone released in the environment interacts with viral and bacterial particles or with surfaces on which they are deposited to disinfect and sanitize the environment.

Advantageously, the device 1 comprises deozonization means 4 configured to deozonize the room subsequently to ozone sanitization. This solution allows quickly reducing the concentration of ozone present in the room as a result of the disinfection treatment.

It is specified that in the context of the present disclosure expressions such as "deozonize", "deozonization", "deozonizing" relate to the process of removing ozone from an air stream.

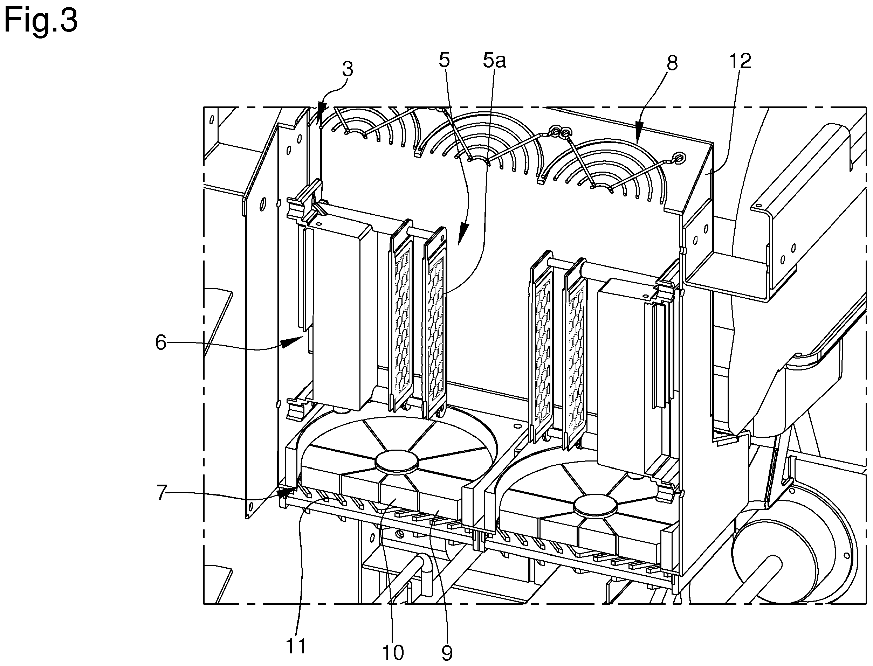

As shown in Figure 3, the sanitizing means 3 comprise an ozone generator 5 and a fluid-operated sanitizing circuit 6 configured to release the ozone generated by the ozone generator 5 into the room. **In** particular, the fluid-operated sanitizing circuit 6 is operationally connected to the ozone generator 5 to take ozone and release it into the environment.

Conveniently, the fluid-operated sanitizing circuit 6 comprises an inlet opening 7 for taking air to be ozonized from the room and an outlet opening 8 for introducing the ozonized air into the room. Moreover, the fluid-operated sanitizing circuit 6 comprises air movement means 9 from the inlet opening 7 to the outlet opening 8. In particular, the air movement means 9 are configured to generate an air stream flowing through the fluid-operated sanitizing circuit 6 from the inlet opening 7 towards the outlet opening 8.

Preferably, the air movement means 9 are of the motorized type, such as a fan 10.

Appropriately, the ozone generator 5 is configured to introduce ozone into the fluid-operated circuit in an inlet area located between the inlet opening 7 and the outlet opening 8, so that the air stream flowing through the fluid-operated sanitizing circuit 6 can mix with the ozone generated by the ozone generator 5 in order to release it into the room.

Preferably, as shown in Figure 3, the ozone generator 5 comprises one or more ozone plates 5a of known type in which the ozone generation occurs by means of the passage of an electrical discharge through a dielectric material.

It cannot however be ruled out to use ozone generators 5 in which ozone is produced by means of different methods.

Conveniently, the fluid-operated sanitizing circuit 6 comprises at least one grid element 11 mounted on the inlet opening 7, and configured to regulate the air stream at inlet. In particular, the grid element 11 has one or more slots of variable size for regulating the air stream at inlet.

As shown in Figure 3, the fluid-operated sanitizing circuit 6 comprises a main duct 12 in which the ozone plate 5a is mounted and at the ends of which the air inlet opening 7 and outlet opening 8 are formed.

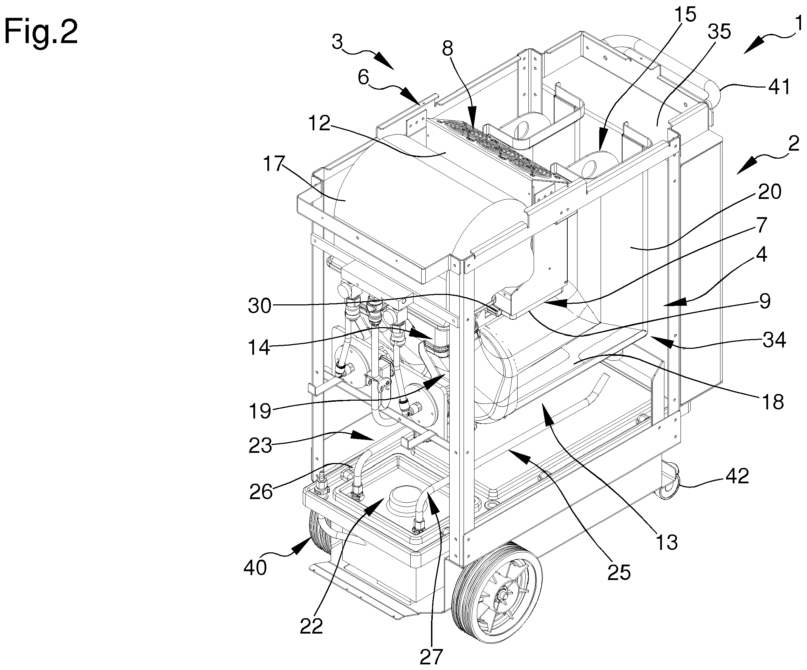

As shown in Figure 2, the duct 12 is mounted in the device 1 in a substantially vertical position with the inlet opening 7 arranged inferiorly to the outlet opening 8.

In the context of the present disclosure, the terms "upper" and "lower", "front", "rear", "vertical" and "horizontal", used with reference to the device 1, are intended to refer to the conditions of normal use of the device 1, i.e. those in which it is placed resting on the ground.

Conveniently, the deozonization means 4 comprise at least one fluid-operated deozonizing circuit 13 provided with an inlet port 14 for taking the air to be deozonized from the room and an outlet port 15 for introducing the deozonized air into the room. Furthermore, the deozonization means 4 comprise filtering means 16, arranged at least partly along the fluid-operated deozonizing circuit 13 and positioned between the inlet port 14 and the outlet port 15, configured to remove ozone from the air to be deozonized.

Advantageously, the fluid-operated sanitizing circuit 6 is separated from the fluid-operated deozonizing circuit 13.

Conveniently, the fluid-operated deozonizing circuit 13 comprises air displacement means 17 from the inlet port 14 to the outlet port 15. In particular, the air displacement means 17 generate an air stream flowing through the fluid-operated deozonizing circuit 13 from the inlet port 14 to the outlet port 15.

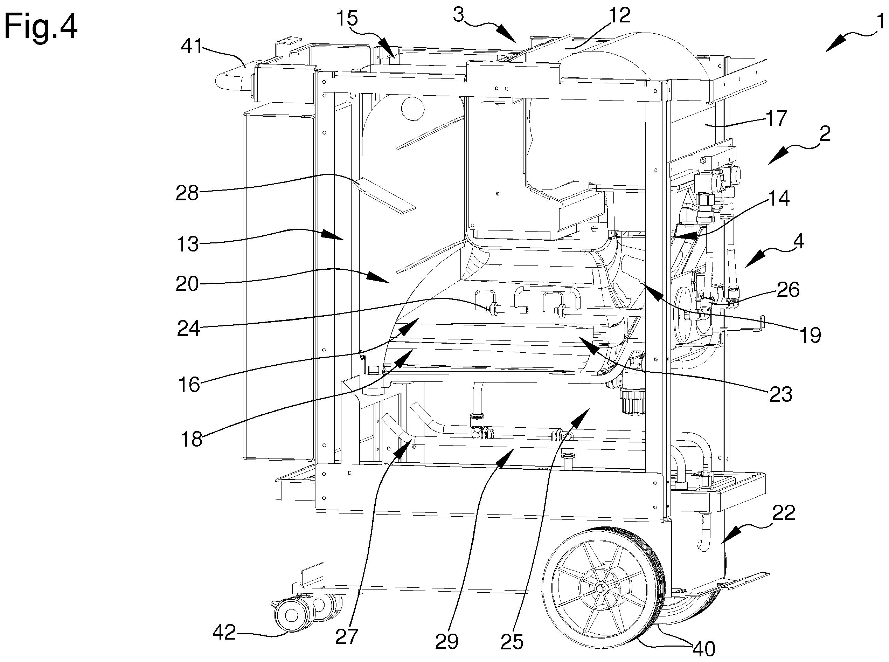

As shown in Figure 4, the fluid-operated deozonizing circuit 13 is provided with a filtering chamber 18 positioned between the inlet port 14 and the outlet port 15, and operationally connected to the filtering means 16. Furthermore, the filtering chamber 18 is connected in a fluid-operated manner to the inlet port 14 and to the outlet port 15 by means of respective inlet ducts 19 and outlet ducts 20.

Preferably, the filtering chamber 18 extends mainly horizontally and the inlet ducts 19 and the outlet ducts 20 are arranged vertically, thereby creating a substantially U-shaped air path in which the at least outlet port 15 faces upwards.

Conveniently, the outlet port 15 of the fluid-operated deozonizing circuit 13 is arranged substantially aligned with the outlet opening 8 of the fluid-operated sanitizing circuit 6. As will be described in detail in the remainder of the present disclosure, this solution allows both fluid-operated circuits 6, 13 to introduce air in the same area of the room.

Appropriately, the filtering means 16 are provided with at least one filtering fluid comprising at least water and adapted to deozonize the air taken from the room.

Preferably, the filtering fluid comprises, in addition to water, one or more detergents.

As shown in Figure 4, the device 1 comprises a tank 22 adapted to contain the filtering fluid, and connected in a fluid-operated manner to the filtering chamber 18.

Usefully, the filtering means 16 comprise a dispensing assembly 23 located between the tank 22 and the filtering chamber 18 and configured to take the filtering fluid from the tank 22 and introduce it into the filtering chamber 18. In particular, the dispensing assembly 23 comprises at least one nozzle 24 for spilling the filtering fluid arranged at least partly inside the filtering chamber 18 and a pumping system 25 for pumping the filtering fluid from the tank 22 to the nozzle 24.

The pumping system 25 is provided with at least one feeding duct 26 of the nozzle 24 and a pump that takes the filtering fluid and feeds it into the feeding duct 26 to send it to the nozzle 24.

Conveniently, the nozzle 24 is configured to feed the filtering fluid into the filtering chamber 18 in a direction opposite to the direction of movement of the air stream inside the fluid-operated deozonizing circuit 13 in order to intercept the air stream and operate on the ozone released therein to deozonize the air.

As shown in Figure 4, the device 1 may comprise a repriming assembly 27 of the filtering fluid.

**In** particular, the repriming assembly 27 is provided with at least one baffle element 28, arranged downstream of the filtering chamber 18 with respect to the air stream in order to separate the deozonized air from the particles of filtering fluid released therein, and a fluid-operated repriming circuit 29 to collect the separated filtering fluid and convey it to the tank 22.

In detail, the baffle element 28 has a substantially flat shape and is arranged in a sloping position inside the outlet duct 20.

Preferably, the repriming assembly 27 comprises a plurality of baffle elements 28 arranged to define a labyrinth-like path for the air stream.

The filtering fluid intercepted by the baffle element 28 falls by gravity into the filtering chamber 18 where it is collected by the fluid-operated repriming circuit 29 to convey it towards the tank 22. For this purpose, the fluid-operated repriming circuit 29 is provided with at least one pipe that puts the bottom of the filtering chamber 18 in fluidic communication with the tank 22.

Advantageously, the repriming assembly 27 also allows the fluid-operated deozonizing circuit 13 to be used for the purpose of filling the tank 22 with the filtering fluid, also allowing the fluid-operated circuits to be flushed easily and quickly. In particular, an operator can insert the filtering fluid and/or the flushing water into the fluid-operated deozonizing circuit 13 through the outlet port 15 so that it can flow until it reaches the tank 22.

Conveniently, the device 1 comprises control means operationally connected to the sanitizing means 3 and to the deozonization means 4 to control at least the activation/deactivation thereof. In particular, the control means make it possible to automate the operation of the device 1 by appropriately programming the sanitizing and deozonization cycles.

Conveniently, the control means are configured to sequentially carry out the following phases:
- activate the sanitizing means 3 to release ozone in the room,
- deactivate the sanitizing means 3,
- activate the deozonization means 4 to deozonize the air of the room,
- deactivate the deozonization means 4.

Preferably, the control means are of the type of an electronic control device such as e.g. a PLC, microcontroller, PC and/or the like.

Conveniently, the control means may be configured to activate the sanitizing means 3 and/or the deozonization means 4 for a predefined activation time, usually based on the size of the room to be sanitized.

For this purpose, the control means comprise an interfacing device by means of which the activation time of the sanitizing means 3 and/or of the deozonization means 4 is settable by the operator.

As shown in Figure 2, the device 1 may be provided with an ozone sensor 30 configured to measure the amount of ozone present in the air of the room. Preferably, the ozone sensor 30 is configured to measure the concentration of ozone present in the air of the room according to one or more known methods. The ozone sensor 30 is operationally connected to the control means in order to provide them with the ozone measurement value.

Appropriately, the control means are configured to selectively control the operation of the sanitizing means 3 and/or of the deozonization means 4 depending on the amount of ozone measured by the ozone sensor 30.

In particular, the control means are configured to deactivate the sanitizing means 3 and activate the deozonization means 4 when the value of the ozone amount measured by the ozone sensor 30 is above a predetermined threshold value. Furthermore, the control means may also be configured to deactivate the deozonization means 4 and activate the sanitizing means 3 when the value of the ozone amount measured by the ozone sensor 30 is below the threshold value.

Conveniently, the device 1 may be provided with a motion sensor configured to detect the presence and/or the passage of a person inside the room in which the device 1 is located.

**In** particular, the motion sensor, when it detects the presence and/or the passage of a person, is configured to interrupt the operation of the sanitizing means 3 and activate the deozonization means 4 to deozonize the air of the room.

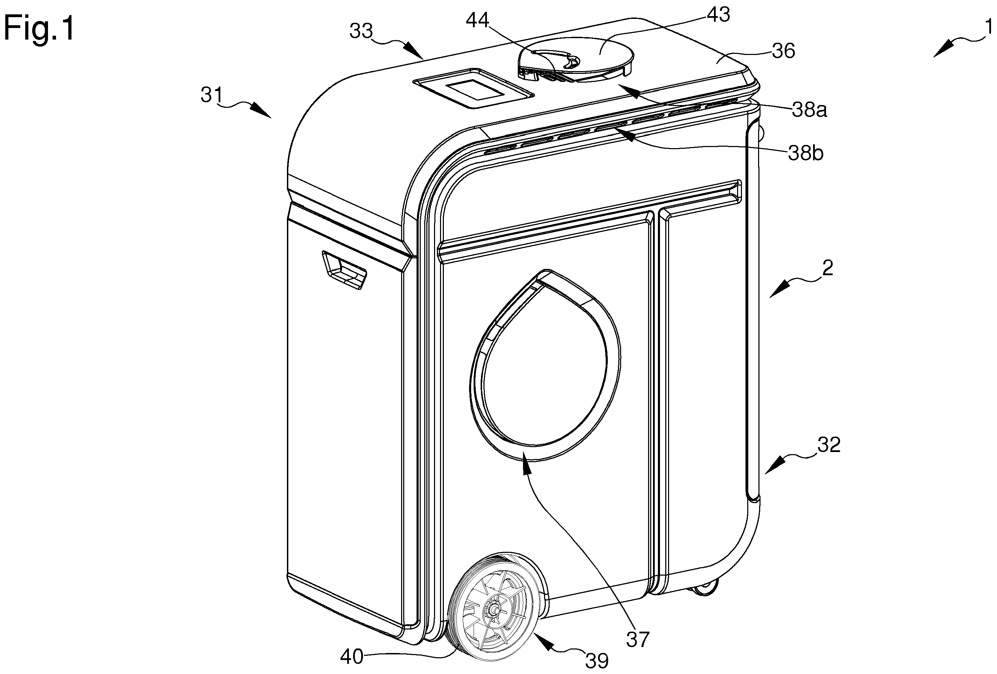

As shown in Figure 1, the supporting frame 2 comprises a casing 31 adapted to enclose at least partly the fluid-operated sanitizing circuit 6 and the fluid-operated deozonizing circuit 13.

Conveniently, the casing 31 has at least one intake chamber 32, wherein the inlet port 14 and the inlet opening 7 of the fluid-operated circuits 6, 13 are facing, and an exhalation chamber 33 wherein the outlet port 15 and the outlet opening 8 are facing.

Conveniently, the intake chambers 31 and the exhalation chambers 33 are separated from each other. This allows the device 1 to take air from an area of the room other than the area into which the air taken in is being fed, thus promoting effective recirculation of air in the room.

As shown in Figure 1, the casing 31 is provided one or more side walls defining a housing compartment 34 of the sanitizing means 3 and deozonization means 4.

The casing 31 also comprises a separator element 35 arranged internally to the housing compartment 34 to define the intake chamber 32 below it and the exhalation chamber 33 above it.

In addition, the casing 31 is provided with a closure lid 36 arranged above the exhalation chamber 33 to close the housing compartment 34.

Appropriately, the casing 31 is provided with at least one inlet slot 37 of air from the room, made on the intake chamber 32, and at least one outlet slot 38a, 38b of air in the room, made on the exhalation chamber 33. In particular, the slots 37, 38a, 38b are pass-through to put in fluidic communication the interior of each chamber 32, 33 with the external environment, thus allowing the fluid-operated circuits 6, 13 to take and feed air from/into the external environment. Preferably, each slot 37, 38a, 38b may be defined as a whole by a series of closely spaced slits.

In one or more embodiments, the casing 31 is provided with a plurality of inlet slots 37 arranged on both sides of the intake chamber 32.

Furthermore, the casing 31 may comprise a pair of outlet slots 38a, 38b. Preferably, the first outlet slot 38a is recessed on the closure lid 36 and the second outlet slot 38b is recessed peripherally around the closure lid 36.

Usefully, the casing 31 comprises a covering element 43 of the first outlet slot 38a. Preferably, the covering element 43 is removable to allow an operator to access at least the outlet port 15 of the fluid-operated deozonizing circuit 13, allowing, as described above, the insertion inside the latter of the filtering fluid and/or of a washing liquid in order to fill the tank 22 and/or to flush the circuit.

Conveniently, the covering element 43 comprises a grid element 44 arranged facing the first outlet slot 38a and adapted to house one or more fragrant substances, such as essential oils, in order to mix them with the air stream exiting the first outlet slot 38a in order to release them in the room during the sanitization treatment.

As shown in Figure 1, the device 1 comprises a ground movement assembly 39 of the supporting frame 2, which allows the device 1 to be transported between different rooms in order to sanitize them.

Preferably, the ground movement assembly 39 comprises a pair of front wheels 40 mounted on the supporting frame 2 and a handle 41 grippable by an operator to move the device 1.

Preferably, the ground movement means 39 comprise one or more rear wheels 42, preferably swivel wheels.

In an alternative embodiment, the device 1 comprises a plurality of deozonization means 4 placed side by side to each other. For example, in the embodiment illustrated in the figures, the device 1 comprises a pair of deozonization means 4.

It goes without saying that the device 1 may be intended to contain a greater or lesser number of deozonization means 4 and/or of sanitizing means 3 depending on operational requirements. In such an embodiment, preferably, the fluid-operated deozonizing circuits 13 of the plurality of deozonization means 4 may be connected to single air displacement means 17.

It is in practice been ascertained that the described invention achieves the intended objects, and in particular the fact is emphasized that, by means of the device according to the present invention, it is possible to sanitize the air of a room in a quick, fast and effective manner, while reducing equally effectively the concentration of ozone present in the air.

## Claims

1. Ozone sanitizing device (1) comprising:
- a supporting frame (2), positionable inside a room to be sanitized;
- ozone sanitizing means (3) mounted on said supporting frame (2) and configured to release ozone into said room;
- deozonization means (4) configured to deozonize said room subsequently to said ozone sanitization;
wherein said deozonization means (4) comprises at least:
- a fluid-operated deozonizing circuit (13) provided with an inlet port (14) for taking the air to be deozonized from said room and an outlet port (15) for introducing the deozonized air into said room,
- filtering means (16), arranged along said fluid-operated deozonizing circuit (13) and positioned between said inlet port (14) and said outlet port (15), configured to remove said ozone from said air to be deozonized;
**characterized by** the fact that said filtering means (16) comprise at least one filtering fluid comprising at least water and adapted to deozonize said air taken from said room.

2. Device (1) according to claim 1, **characterized by** the fact that said ozone sanitizing means (3) comprise an ozone generator (5) and a fluid-operated sanitizing circuit (6) configured to release said ozone generated by said ozone generator (5) into said room.

3. Device (1) according to the preceding claim, **characterized by** the fact that said fluid-operated sanitizing circuit (6) comprises an inlet opening (7) for taking air to be ozonized from said room and an outlet opening (8) for introducing the ozonized air into said room, said ozone generator (5) being configured to introduce ozone in said fluid-operated sanitizing circuit (6) in an input area positioned between said inlet opening (7) and said outlet opening (8).

4. Device (1) according to claim 2 or 3, **characterized by** the fact that said fluid-operated sanitizing circuit (6) is separated from said fluid-operated deozonizing circuit (13).

5. Device (1) according to one or more of the preceding claims, **characterized by** the fact that it comprises control means operationally connected to said ozone sanitizing means (3) and to said deozonization means (4) to selectively control at least the activation/deactivation thereof.

6. Device (1) according to the preceding claim, **characterized by** the fact that said control means are configured to sequentially carry out the following phases:
- activate said ozone sanitizing means (3) to release said ozone in said room,
- deactivate said ozone sanitizing means (3),
- activate said deozonization means (4) to deozonize the air of said room,
- deactivate said deozonization means (4).

7. Device (1) according to one or more of the preceding claims, **characterized by** the fact that it comprises an ozone sensor (30) configured to measure the amount of ozone present in the air of said room.

8. Device (1) according to claim7 and claim 5 or 6, **characterized by** the fact that said control means are configured to selectively control the operation of said sanitizing means (3) and/or said deozonization means (4) depending on said amount of ozone measured by said ozone sensor (30).

9. Device (1) according to claim 3, **characterized by** the fact that said supporting frame (2) comprises a casing (31) adapted to enclose at least partly said fluid-operated sanitizing circuit (6) and said fluid-operated deozonizing circuit (13),
and by the fact that
said casing (31) defines at least one intake chamber (32), wherein are facing said inlet port (14) and said inlet opening (7), and an exhalation chamber (33) wherein are facing said outlet port (15) and said outlet opening (8).

10. Device (1) according to claim 9, **characterized by** the fact that said casing (31) comprises at least one air inlet slot (37) from said room made on said intake chamber (32) and at least one air outlet slot (38a, 38b) in said room made on said exhalation chamber (33), said inlet and outlet slots (37, 38a, 38b) being adapted to set said room and said intake and exhalation chambers (32, 33) in fluidic communication to each other.

## Patentansprüche

1. Ozon-Desinfektionsvorrichtung (1) umfassend:
- einen Tragrahmen (2), der in einem zu desinfizierenden Raum positionierbar ist;
- Ozon-Desinfektionsmittel (3), die an dem Tragrahmen (2) angebracht und ausgebildet sind, Ozon in den Raum abzugeben;
- Deozonisierungsmittel (4), die ausgebildet sind, den Raum nach der Desinfektion mit Ozon von Ozon zu befreien;
wobei die Deozonisierungsmittel (4) mindestens Folgendes umfassen:
- einen fluidbetriebenen Deozonisierungskreislauf (13) mit einem Einlassanschluss (14) zur Aufnahme der zu deozonisierenden Luft aus dem Raum und einem Auslassanschluss (15) zum Einleiten der deozonisierten Luft in den Raum,
- Filtermittel (16), die entlang des fluidbetriebenen Deozonisierungskreislaufs (13) angeordnet und zwischen dem Einlassanschluss (14) und dem Auslassanschluss (15) positioniert und ausgebildet sind, das Ozon aus der zu deozonisierenden Luft zu entfernen;
**dadurch gekennzeichnet, dass** die Filtermittel (16) mindestens ein Filterfluid umfassen, das mindestens Wasser enthält und ausgebildet ist, die aus dem Raum entnommene Luft zu deozonisieren.

2. Vorrichtung (1) gemäß Anspruch 1, **dadurch gekennzeichnet, dass** die Ozon-Desinfektionsmittel (3) einen Ozongenerator (5) und einen fluidbetriebenen Desinfektionskreislauf (6) umfassen, der ausgebildet ist, das von dem Ozongenerator (5) erzeugte Ozon in den Raum abzugeben.

3. Vorrichtung (1) gemäß dem vorhergehenden Anspruch, **dadurch gekennzeichnet, dass** der fluidbetriebenen Desinfektionskreislauf (6) eine Einlassöffnung (7) zur Aufnahme der zu ozonisierenden Luft aus dem Raum und eine Auslassöffnung (8) zum Einleiten der ozonisierten Luft in den Raum umfasst, wobei der Ozongenerator (5) ausgebildet ist, Ozon in den fluidbetriebenen Desinfektionskreislauf (6) in einem zwischen der Einlassöffnung (7) und der Auslassöffnung (8) angeordneten Einlassbereich einzuleiten.

4. Vorrichtung (1) gemäß Anspruch 2 oder 3, **dadurch gekennzeichnet, dass** der fluidbetriebenen Desinfektionskreislauf (6) von dem fluidbetriebenen Deozonisierungskreislauf (13) getrennt ist.

5. Vorrichtung (1) gemäß einem oder mehreren der vorhergehenden Ansprüche, **dadurch gekennzeichnet, dass** sie Steuermittel umfasst, die mit den Ozon-Desinfektionsmitteln (3) und den Deozonisierungsmitteln (4) wirkverbunden sind, um zumindest deren Aktivierung/Deaktivierung selektiv zu steuern.

6. Vorrichtung (1) gemäß dem vorhergehenden Anspruch, **dadurch gekennzeichnet, dass** die Steuermittel ausgebildet sind, nacheinander die folgenden Phasen ausführen:
- Aktivieren der Ozon-Desinfektionsmittel (3), um das Ozon in den Raum abzugeben,
- Deaktivieren der Ozon-Desinfektionsmittel (3),
- Aktivieren der Deozonisierungsmittel (4), um die Luft des Raums zu deozonisieren,
- Deaktivieren der Deozonisierungsmittel (4).

7. Vorrichtung (1) gemäß einem oder mehreren der vorhergehenden Ansprüche, **dadurch gekennzeichnet, dass** sie einen Ozonsensor (30) umfasst, der ausgebildet ist, die in der Luft des Raums vorhandene Ozonmenge zu messen.

8. Vorrichtung (1) gemäß Anspruch 7 und Anspruch 5 oder 6, **dadurch gekennzeichnet, dass** die Steuermittel ausgebildet sind, den Betrieb der Desinfektionsmittel (3) und/oder der Deozonisierungsmittel (4) in Abhängigkeit von der von dem Ozonsensor (30) gemessenen Ozonmenge selektiv zu steuern.

9. Vorrichtung (1) gemäß Anspruch 3, **dadurch gekennzeichnet, dass** der Tragrahmen (2) ein Gehäuse (31) umfasst, das ausgebildet ist, den fluidbetriebenen Desinfektionskreislauf (6) und den fluidbetriebenen Deozonisierungskreislauf (13) zumindest teilweise zu umschließen, und dadurch, dass das Gehäuse (31) mindestens eine Einlasskammer (32), in die der Einlassanschluss (14) und die Einlassöffnung (7) münden, und eine Auslasskammer (33) definiert, in die der Auslassanschluss (15) und die Auslassöffnung (8) münden.

10. Vorrichtung (1) gemäß Anspruch 9, **dadurch gekennzeichnet, dass** das Gehäuse (31) mindestens einen an der Einlasskammer (32) ausgebildeten Lufteinlassschlitz (37) von dem Raum aus aufweist, und mindestens einen an der Auslasskammer (33) ausgebildeten Luftauslassschlitz (38a, 38b) in den Raum umfasst, wobei die Einlass- und Auslassschlitze (37, 38a, 38b) ausgebildet sind, den Raum und die Einlass- und Auslasskammern (32, 33) in Fluidverbindung miteinander zu bringen.

## Revendications

1. - Dispositif de désinfection à l'ozone (1) comprenant :
- un cadre de support (2), positionnable à l'intérieur d'une pièce de désinfecter ;
- des moyens de désinfection à l'ozone (3) montés sur ledit cadre de support (2) et configurés pour libérer de l'ozone dans ladite pièce ;
- des moyens de dé-ozonation (4) configurés pour dé-ozoner ladite pièce après ladite désinfection à l'ozone ;
dans lequel lesdits moyens de dé-ozonation (4) comprennent au moins :
- un circuit de dé-ozonation actionné par fluide (13) muni d'un orifice d'entrée (14) pour prélever l'air à dé-ozoner dans ladite pièce et d'un orifice de sortie (15) pour introduire l'air dé-ozoné dans ladite pièce ;
- des moyens de filtration (16), disposés le long dudit circuit de dé-ozonation actionné par fluide (13) et positionnés entre ledit orifice d'entrée (14) et ledit orifice de sortie (15), configurés pour retirer ledit ozone de l'air à dé-ozoner ;
**caractérisé par le fait que** lesdits moyens de filtration (16) comprennent au moins un fluide filtrant comprenant au moins de l'eau et apte à dé-ozoner ledit air prélevé dans ladite pièce.

2. - Dispositif (1) selon la revendication 1, **caractérisé par le fait que** lesdits moyens de désinfection à l'ozone (3) comprennent un générateur d'ozone (5) et un circuit de désinfection actionné par fluide (6) configuré pour libérer ledit ozone généré par ledit générateur d'ozone (5) dans ladite pièce.

3. - Dispositif (1) selon la revendication précédente, **caractérisé par le fait que** ledit circuit de désinfection actionné par fluide (6) comprend une ouverture d'entrée (7) pour prélever l'air à ozoner dans ladite pièce et une ouverture de sortie (8) pour introduire l'air ozoné dans ladite pièce, ledit générateur d'ozone (5) étant configuré pour introduire de l'ozone dans ledit circuit de désinfection actionné par fluide (6) dans une zone d'entrée positionnée entre ladite ouverture d'entrée (7) et ladite ouverture de sortie (8).

4. Dispositif (1) selon l'une des revendications 2 ou 3, **caractérisé par le fait que** ledit circuit de désinfection actionné par fluide (6) est séparé dudit circuit de dé-ozonation actionné par fluide (13).

5. - Dispositif (1) selon l'une ou plusieurs des revendications précédentes, **caractérisé par le fait qu'**il comprend des moyens de commande reliés fonctionnellement auxdits moyens de désinfection à l'ozone (3) et auxdits moyens de dé-ozonation (4) pour en commander sélectivement au moins l'activation/la désactivation.

6. - Dispositif (1) selon la revendication précédente, **caractérisé par le fait que** lesdits moyens de commande sont configurés pour effectuer séquentiellement les phases suivantes :
- activer lesdits moyens de désinfection à l'ozone (3) pour libérer ledit ozone dans ladite pièce ;
- désactiver lesdits moyens de désinfection (3) ;
- activer lesdits moyens de dé-ozonation (4) pour dé-ozoner l'air de ladite pièce ;
- désactiver lesdits moyens de dé-ozonation (4).

7. - Dispositif (1) selon l'une ou plusieurs des revendications précédentes, **caractérisé par le fait qu'**il comprend un capteur d'ozone (30) configuré pour mesurer la quantité d'ozone présent dans l'air de ladite pièce.

8. - Dispositif (1) selon la revendication 7 et l'une des revendications 5 ou 6, **caractérisé par le fait que** lesdits moyens de commande sont configurés pour commander sélectivement le fonctionnement desdits moyens de désinfection (3) et/ou desdits moyens de dé-ozonation (4) en fonction de ladite quantité d'ozone mesurée par ledit capteur d'ozone (30).

9. - Dispositif (1) selon la revendication 3, **caractérisé par le fait que** ledit cadre de support (2) comprend un carter (31) apte à renfermer au moins partiellement ledit circuit de désinfection commandé par fluide (6) et ledit circuit de dé-ozonation actionné par fluide (13),
et **par le fait que**
ledit carter (31) définit au moins une chambre d'admission (32), dans laquelle donnent ledit orifice d'entrée (14) et ladite ouverture d'entrée (7), et une chambre d'émission (33) dans laquelle donnent ledit orifice de sortie (15) et ladite ouverture de sortie (8).

10. - Dispositif (1) selon la revendication 9, **caractérisé par le fait que** ledit carter (31) comprend au moins une fente d'entrée d'air (37) de ladite pièce réalisée sur ladite chambre d'admission (32) et au moins une fente de sortie d'air (38a, 38b) dans ladite pièce réalisée sur ladite chambre d'émission (33), lesdites fentes d'entrée et de sortie (37, 38a, 38b) étant aptes à mettre ladite pièce et lesdites chambres d'admission et d'émission (32, 33) en communication fluidique l'une avec l'autre.
